# EUROPEAN PATENT APPLICATION

(11) **EP 2 557 415 A1**
(43) Date of publication of application: **13.02.2013**
(21) Application number: 12179624.7
(22) Date of filing: 08.08.2012
(51) Int. Cl.: G01N 21/64, A61B 5/145

(54) **Fluorescence sensor**

(30) Priority: 11.08.2011 JP 2011176257
(71) Applicant: OLYMPUS CORPORATION, Shibuya-ku Tokyo 151-0072 (JP); Terumo Kabushiki Kaisha, Tokyo 151-0072 (JP)
(72) Inventor: Maeda, Kazuya, Tokyo Tokyo 151-0072 (JP); Matsumoto, Atsushi, Kanagawa 259-0151 (JP)
(74) Representative: Winter, Brandl, Fürniss, Hübner, Röss, Kaiser, Polte - Partnerschaft

(57) **Abstract**

A fluorescence sensor 10 includes a silicon substrate 11 on which a PD element 12 that converts fluorescence into an electric signal is formed, an LED substrate 15 having a first principal plane 15A on which an LED element 15C that generates excitation light is formed, a reflective film 20 that averages a light amount distribution of the excitation light radiated from a second principal plane 15B of the LED substrate 15, and an indicator layer 17 that receives the excitation light averaged by the reflective film 20 and generates the fluorescence having a light amount corresponding to an analyte amount.

## Description

### BACKGROUND OF THE INVENTION

### 1. Field of the Invention

The present invention relates to a fluorescence sensor that measures analyte density and, more particularly, to a fluorescence sensor including an indicator that generates, according to irradiation by excitation light, fluorescence having a light amount corresponding to analyte density.

### 2. Description of the Related Art

Various analyzers have been developed in order to measure density of an analyte, i.e., a substance to be measured in liquid. For example, a fluorescence sensor is known that measures analyte density by injecting a fluorescent coloring matter, which changes in characteristics according to presence of the analyte and generates fluorescence, and a solution to be measured including the analyte into a transparent container having a fixed capacity, irradiating the solution with excitation light, and measuring intensity of the fluorescence from the fluorescent coloring matter.

A small fluorescence sensor includes a light source, a photodetector, and an indicator layer containing a fluorescent coloring matter. The fluorescence sensor irradiates the indicator layer, into which analyte in a solution to be measured can penetrate, with excitation light from the light source. Then, the fluorescent coloring matter in the indicator layer generates fluorescence having a light amount corresponding to analyte density in the solution to be measured. The photodetector receives the fluorescence. The photodetector is a photoelectric conversion element and outputs an electric signal corresponding to the received light amount. The analyte density in the solution to be measured is measured from the electric signal.

In recent years, in order to measure analyte in a very small amount of a sample, a fluorescence sensor is proposed that is manufactured using a semiconductor manufacturing technique and a micro machine manufacturing technique.

For example, a fluorescence sensor 110 shown in Figs. 1 and 2 is disclosed in the specification of United States Patent No. 5039490. Fig. 1 shows a schematic sectional structure of the fluorescence sensor 110. Fig. 2 is a disassembled view for explaining a schematic structure of the fluorescence sensor 110. In figures referred to below, analyte 2 is schematically shown.

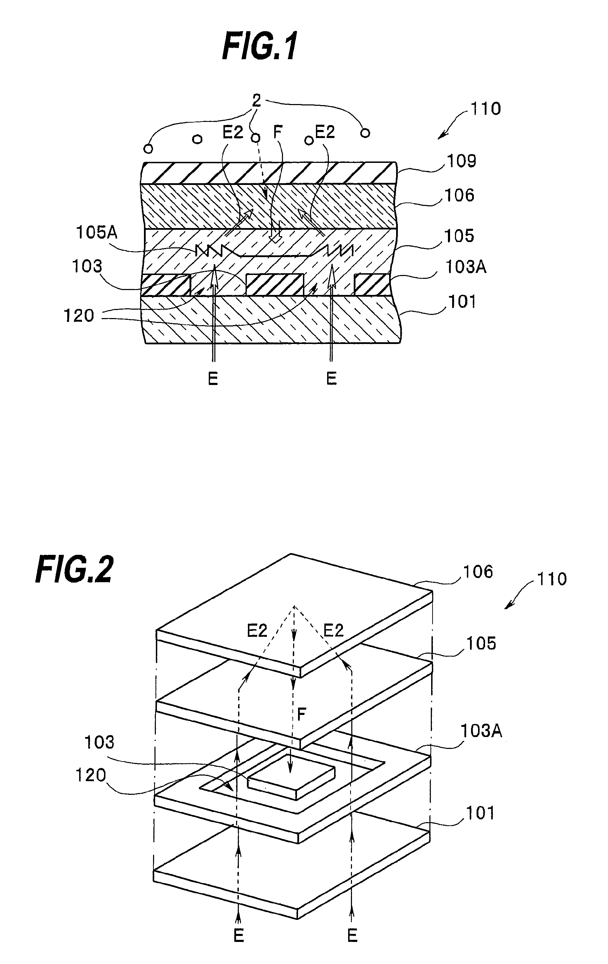

As shown in Figs. 1 and 2, the fluorescence sensor 110 includes a transparent supporting substrate 101 through which excitation light E can be transmitted, photoelectric conversion element sections 103 that convert fluorescence F into an electric signal, an optical tabular section 105 including a condensing function section 105A that condenses the excitation light E, an indicator layer 106 that mutually acts with the analyte 2 to thereby emit the fluorescence F according to incidence of the excitation light E, and a cover layer 109.

In the photoelectric conversion element sections 103, photoelectric conversion elements are formed on substrates 103A made of, for example, silicon. The substrates 103A do not transmit the excitation light E. Therefore, the fluorescence sensor 110 includes gap regions 120, through which the excitation light E can be transmitted, around the photoelectric conversion element sections 103.

This means that only the excitation light E transmitted through the gap regions 120 and made incident on the optical tabular section 105 is condensed near upper parts of the photoelectric conversion element sections 103 in the indicator layer 106 according to the action of the optical tabular section 105. The fluorescence F is generated by mutual action of the condensed excitation light E2 and the analyte 2 penetrating into an inside of the indicator layer 106. A part of the generated fluorescence F is made incident on the photoelectric conversion element sections 103. In the photoelectric conversion element sections 103, a signal of an electric current, a voltage, or the like proportional to fluorescence intensity, i.e., density of the analyte 2 is generated. The excitation light E is not made incident on the photoelectric conversion element section 103 according to the action of filters (not shown) formed on the photoelectric conversion element sections 103.

In the fluorescence sensor 110, the excitation light E is condensed near the upper parts of the photoelectric conversion element sections 103 of the indicator layer 106. Therefore, it is likely that an indicator in a condensing place is deteriorated fast and life of the entire sensor is reduced.

It is an object of the present invention to provide a fluorescence sensor having long life.

### SUMMARY OF THE INVENTION

A fluorescence sensor according to an embodiment includes: a main substrate on which a photoelectric conversion element that converts fluorescence into an electric signal is formed; a light-emitting element substrate having a first principal plane on which a light-emitting element that generates excitation light is formed; a light amount uniformizing section that uniformizes a light amount distribution of the excitation light radiated from a second principal plane of the light-emitting element substrate; and an indicator that receives the excitation light uniformized by the light amount uniformizing section and generates the fluorescence having a light amount corresponding to an analyte amount.

### BRIEF DESCRIPTION OF THE DRAWINGS

Fig. 1 is an explanatory diagram showing a schematic sectional structure of a fluorescence sensor in the past;
Fig. 2 is a disassembled view for explaining a schematic structure of the fluorescence sensor in the past;
Fig. 3 is a schematic diagram showing a schematic sectional structure of a fluorescence sensor according to a first embodiment;
Fig. 4 is a disassembled view for explaining a schematic structure of the fluorescence sensor according to the first embodiment;
Fig. 5A is an explanatory diagram for explaining a reflective film according to the first embodiment;
Fig. 5B is an explanatory diagram for explaining the reflective film according to the first embodiment;
Fig. 5C is an explanatory diagram for explaining the reflective film according to the first embodiment;
Fig. 5D is an explanatory diagram for explaining the reflective film according to the first embodiment;
Fig. 6 is an explanatory diagram for explaining an optical path of excitation light in the fluorescence sensor according to the first embodiment;
Fig. 7 is an explanatory diagram for explaining a light amount distribution change due to the reflective film according to the first embodiment;
Fig. 8 is an explanatory diagram for explaining an optical path of excitation light in a fluorescence sensor according to a modification of the first embodiment;
Fig. 9 is an explanatory diagram for explaining a light amount distribution change due to a light blocking film according to a second embodiment;
Fig. 10 is a schematic diagram showing a schematic sectional structure of a fluorescence sensor according to a third embodiment; and
Fig. 11 is a schematic diagram showing a schematic sectional structure of a fluorescence sensor according to a modification of the third embodiment.

### DETAILED DESCRIPTION OF THE PREFERRED EMBODIMENTS

### First Embodiment

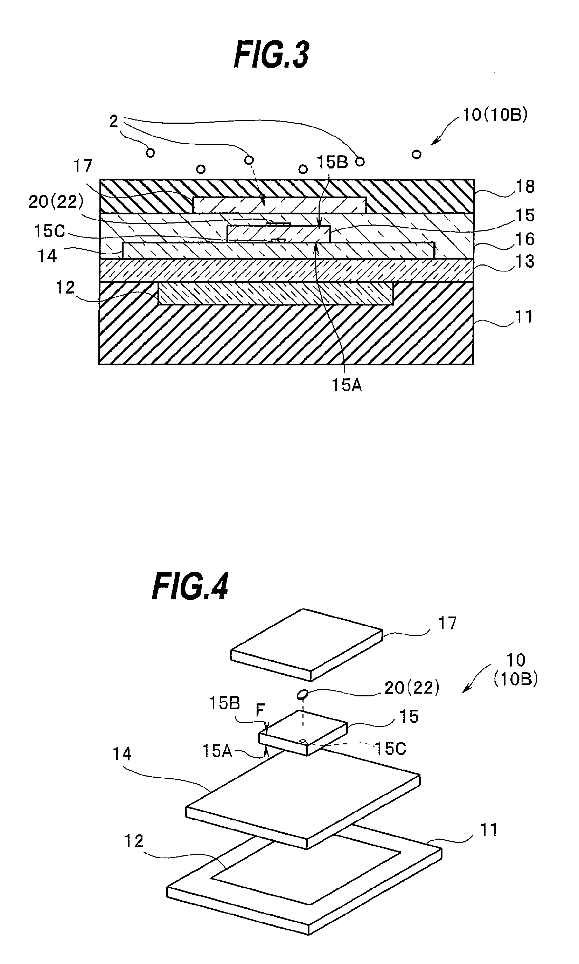

A fluorescence sensor 10 according to a first embodiment of the present invention is explained below with reference to the accompanying drawings. As shown in Figs. 3 and 4, the fluorescence sensor 10 according to the present embodiment has structure including a silicon substrate 11 functioning as a main substrate, a silicon oxide film 13, a filter 14, a light-emitting element substrate (hereinafter referred to as "LED substrate") 15 on which a reflective film 20 functioning as a light amount uniformizing section that uniformizes a light amount distribution of radiated excitation light is disposed, a transparent resin layer 16, an indicator layer 17, and a light blocking layer 18, which are laminated in order from the silicon substrate 11 side. On the silicon substrate 11, a photodiode element (hereinafter referred to as "PD element") 12 functioning as a photoelectric conversion element is formed. In substantially a center of a first principal plane 15A of the LED substrate 15 that transmits excitation light E and fluorescence F, a light-emitting diode element (hereinafter referred to as "LED element") 15C functioning as a light-emitting element that generates the excitation light E is formed. Wires and the like connected to the PD element 12 and the like are not shown in the figures.

As explained later, the reflective film 20 is a dielectric multilayer film that is disposed in substantially a center of a second principal plane 15B of the LED substrate 15 and reflects the excitation light E and transmits the fluorescence F. The filter 14 blocks the excitation light E and transmits the fluorescence F having wavelength longer than wavelength of the excitation light E.

At least a part of the PD element 12, the filter 14, the LED element 15C, and the indicator layer 17 are formed in the same region on the silicon substrate 11. In the fluorescence sensor 10, it is desirable that centers of the PD element 12, the filter 14, the LED element 15C, and the indicator layer 17 are formed in the same region on the silicon substrate 11.

In short, in the fluorescence sensor 10, structure totally different from the fluorescence sensor in the past explained above is realized by using the LED substrate 15 that transmits the fluorescence F from the indicator layer 17.

The silicon substrate 11 is a main substrate having a surface on which the PD element 12 is formed. When the PD element 12 is formed on the substrate surface as the photoelectric conversion element, a monocrystal silicon substrate is suitable as the main substrate. However, depending on a manufacturing method for the PD element 12, the main substrate can be selected out of various materials such as a glass substrate.

The PD element 12 is the photoelectric conversion element that converts fluorescence into an electric signal. The photoelectric conversion element can be selected out of various photoelectric conversion elements such as a photoconductor and a phototransistor (PT). A photodiode or the phototransistor is particularly desirable because fluorescence detection sensitivity highest and excellent in stability can be realized and, as a result, the fluorescence sensor 10 excellent in detection sensitivity and detection accuracy can be realized.

The silicon oxide film 13 is a first protective film. As the first protective film having thickness of, for example, several tens to several hundreds nanometers, a silicon nitride film or a composite laminated film including a silicon oxide film and the silicon nitride film may be used.

The filter 14 has transmittance selectivity equal to or larger than five digits as a ratio of transmittance that depends on wavelength, for example, transmittance equal to or lower than 10-6 at wavelength of the excitation light E shorter than 375 nm and transmittance equal to or higher than 10-1, i.e., 10% at wavelength of the fluorescence F equal to 460 nm. The filter 14 may be a reflective filter same as the reflective film 20 or may be an absorption type filter including a silicon film, a silicon carbide film, or the like.

The LED substrate 15 is a substrate on which the LED element 15C can be formed and that transmits the fluorescence F, for example, a sapphire substrate. The sapphire substrate has high transmittance of the fluorescence F. On the sapphire substrate, an LED element 15C that is made of a gallium nitride compound semiconductor and emits ultraviolet ray can be formed.

The LED element 15C is formed substantially in the center of the first principal plane 15A of the LED substrate 15. Excitation light passes through an inside of the LED substrate 15 and is radiated from the second principal plane 15B. A light amount of the excitation light E radiated from the second principal plane 15B has a predetermined distribution in which the light amount is large in a center region opposed to the LED element 15C and small in a peripheral region.

The reflective film 20 is a reflective filter having an interference effect in which a high refractive index layer and a low refractive index layer having a refractive index lower than a refractive index of the high refractive index layer are alternately laminated. For example, after being formed over the entire surface of the second principal plane 15B, the reflective film 20 is patterned into a desired shape by masking a desired region and then removing an unnecessary region with etching or the like. Or the reflective film 20 may be disposed on the second principal plane 15B after being formed in a desired pattern.

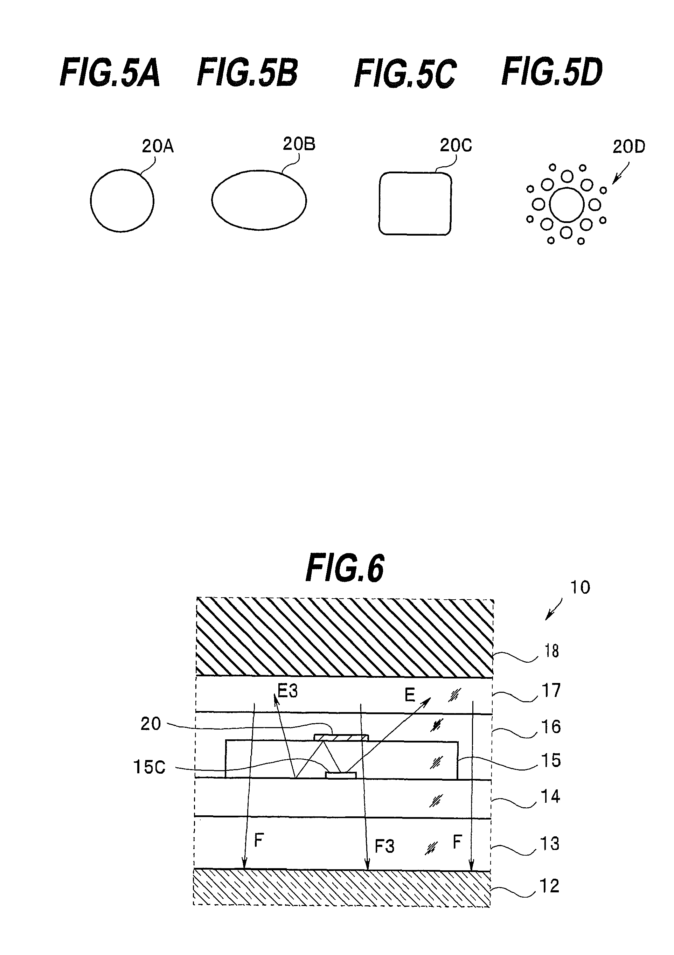

As shown in Figs. 5A to 5D, the reflective film 20 may include one pattern such as a circular pattern 20A (Fig. 5A), an elliptical pattern 20B (Fig. 5B), or a rectangular pattern 20C (Fig. 5C) or may include plural patterns 20D (Fig. 5D).

An epoxy resin film functioning as the transparent resin layer 16 is a second protective film. As the second protective film, for example, silicone resin, transparent amorphous fluorine resin, or the like can also be used.

The indicator layer 17 generates fluorescence having a light amount corresponding to density of the analyte 2 according to mutual action with the penetrating analyte 2 and the excitation light. The thickness of the indicator layer 17 is set to about 10 µm to 500 µm, more preferably, to 40 µm to 200 µm. The indicator layer 17 is formed of a base material including a fluorescent coloring material that generates fluorescence having intensity corresponding to an amount of the analyte 2, i.e., analyte density in a sample. The base material of the indicator layer 17 desirably has transparency for allowing the excitation light from the LED element 15C and the fluorescence from the fluorescent coloring material to be satisfactorily transmitted. The fluorescent coloring material may be the analyte 2 itself present in the sample. An indicator may be gel-like or liquid-like rather than being layer-like or film-like.

The fluorescent coloring material is selected according to a type of the analyte 2. Any fluorescent coloring material can be used as long as a light amount of fluorescent generated by the fluorescent coloring material reversibly changes according to an amount of the analyte 2. For example, when hydrogen ion density or carbon dioxide in a living body is measured, a hydroxypyrene trisulfonic acid derivative can be used. When saccharides are measured, a phenyl boron acid derivative having a fluorescence residue can be used. When potassium ions are measured, a crown ether derivative or the like having a fluorescence residue can be used.

When saccharides such as glucose are measured, as the fluorescent coloring material, a ruthenium organic complex, a fluorescent phenyl boron acid derivative, or a substance reversibly combined with glucose such as fluorescein combined with protein can be used. As the ruthenium organic complex, complexes of ruthenium and 2,2'-bipyridine, 1,10-phenanthroline, 4,7-diphenyl-1,10-phenanthroline, 4,7-dimethyl-1,10-phenanthroline, 4,7-disulfonated diphenyl-1,10-phenanthroline, 2,2'-bi-2-thiazoline, 2,2'-bithiazole, 5-bromo-1,10-phenanthroline, 5-chloro-1,10-phenanthroline, and the like can be used. Further, organic complexes containing osmium, iridium, rhodium, rhenium, chrome, and the like instead of ruthenium of the ruthenium organic complex can be used. As fluorescent phenyl boron acid derivative, in particular, a compound containing two phenyl boron acids and containing anthracene as a florescence residue has high detection sensitivity.

As explained above, the fluorescence sensor 10 according to the present invention is adapted to various applications such as an oxygen sensor, a glucose sensor, a pH sensor, an immunity sensor, and a microorganism sensor according to selection of a fluorescent coloring material.

In the indicator layer 17, for example, a hydrogel that is easily impregnated with water is used as a base material and the fluorescent coloring material is contained or combined in the hydrogel. As a component of the hydrogel, an acrylic hydrogel produced by polymerizing a polysaccharide such as methyl cellulose or dextran or a monomer such as (meta) acrylamide, methylolacrylamide, or hydroxyethyl acrylate, a urethane hydrogel produced from polyethylene glycol and diisocyanate, and the like can be used.

The light blocking layer 18 provided as a top layer is a layer having thickness of equal to or smaller than several tens micrometers formed on an upper surface side of the indicator layer 17. The light blocking layer 18 prevents excitation light and fluorescence from leaking to an outside of the fluorescence sensor 10 and, at the same time, prevents external light from penetrating into an inside of the fluorescence sensor 10.

The light blocking layer 18 desirably covers not only the indicator layer 17 but also the entire fluorescence sensor 10 in order to block the external light. The light blocking layer 18 is formed of a material that does not prevent the analyte 2 from passing through an inside of the light blocking layer 18 and reaching the indicator layer 17. In the case of the fluorescence sensor 10 used for an analysis of analyte in a water solution, as the material of the light blocking layer 18, for example, microporous metal or ceramics or a composite material obtained by mixing particulates that do not allow light to pass such as carbon black or carbon nanotube in the hydrogel used in the indicator layer 17 is suitable. The light blocking layer 18 may be a silicon substrate or the like including a large number of through-holes.

The LED substrate 15, the transparent resin layer 16, and the indicator layer 17 may be housed on an inside of a frame-shaped sensor frame. It is possible to prevent penetration of external light and realize improvement of mechanical strength of the entire sensor by forming the sensor frame with a light blocking material such as silicon.

In the fluorescence sensor 10 having the structure explained above, the fluorescent coloring material in the indicator layer 17 is irradiated with the excitation light E from the LED element 15C. The fluorescence F generated by the fluorescent coloring material passes through the LED substrate 15 and the filter 14, reaches the PD element 12, and is converted into an electric signal.

As shown in Fig. 6, in the fluorescence sensor 10, excitation light E3 in a center region having a large light amount on the second principal plane 15B of the LED substrate 15 is reflected by the reflective film 20 and is not directly made incident on the indicator layer 17. The excitation light E3 reflected by the reflective film 20 is further reflected on an inner surface of the LED substrate 15, surfaces of members around the LED substrate 15, and the like. At least a part of the excitation light E3 is made incident on the indicator layer 17.

The fluorescence F generated by the indicator layer 17 is transmitted through the reflective film 20 and the LED substrate 15 and made incident on the PD element 12. Specifically, since fluorescence F3 is transmitted through the reflective film 20 and made incident on the PD element 12, the reflective film 20 does not obstruct detection of fluorescence. Therefore, the sensitivity of the fluorescence sensor 10 does not fall even if the fluorescence sensor 10 includes the reflective film 20 in a passing route of the fluorescence F.

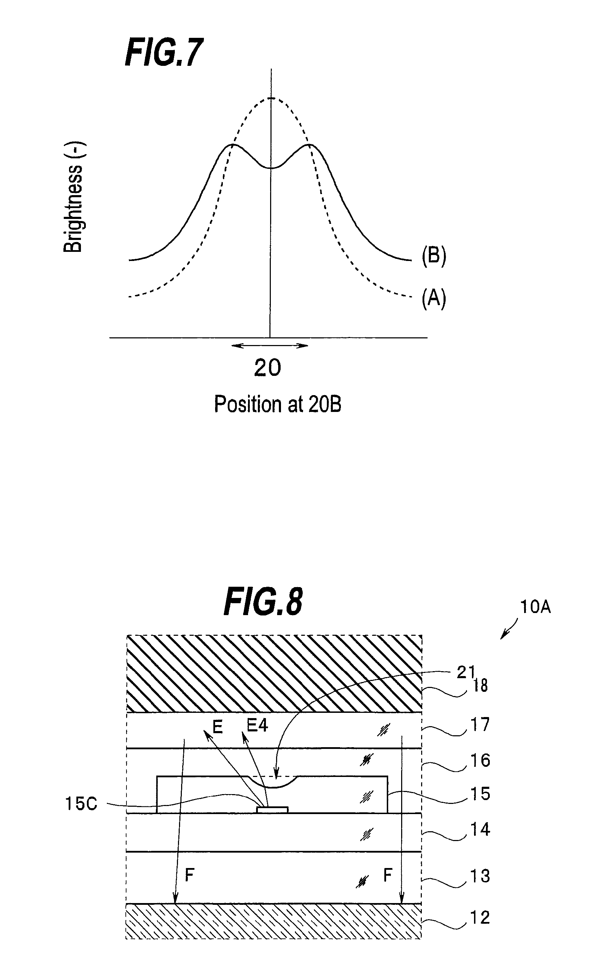

As explained above, the reflective film 20 is the light amount uniformizing section that uniformizes a light amount distribution of the excitation light E radiated from the second principal plane 15B. Fig. 7 is a diagram for explaining a light amount distribution of the excitation light E radiated from the second principal plane 15B. An abscissa center is the center of the second principal plane 15B. As shown in Fig. 7, compared with a case (A) in which the reflective film is absent, in a case (B) in which the reflective film is present, a light amount in a center region decreases. However, a light in a peripheral region increases because a part of light reflected by the reflective film 20 is emitted. In other words, the reflective film 20 is an excitation light diffusing section that averages a light amount distribution of excitation light.

In the fluorescence sensor 10, since a center region of the indicator layer 17 is not concentratedly irradiated with intense excitation light, the indicator is deteriorated slowly. Therefore, the fluorescence sensor has a long life because a problem such as a fall in detection sensitivity less easily occurs for a long period.

The reflective film 20 of the fluorescence sensor 10 is the excitation light diffusing section that not only reduces the excitation light E in a region where a light amount is large but also increases the excitation light E in a region where a light amount is small. Therefore, efficiency of use of the excitation light is high. Therefore, since an electric current fed to the LED element 15C can be suppressed, heat generation of the LED element 15C is small and characteristics of the fluorescence sensor 10 are stable.

### Modification of the First Embodiment

Even in a fluorescence sensor including, instead of the reflective film 20 of the fluorescence sensor 10, a metal film or the like of aluminum or the like that reflects both excitation light and fluorescence as the light amount uniformizing section having the reflecting function, the life improvement effect can be obtained. The metal film or the like is easily formed and inexpensive compared with the multilayer interference film.

A light amount uniformizing section having a diffracting function, a scattering function, or a refracting function can also be used.

The light amount uniformizing section having the diffracting function includes a diffraction grating structure and diffuses incident light according to a diffraction phenomenon. A layout of patterns of the diffraction grating may be any patterns as long as the diffraction grating can cause diffraction of light. For example, parallel lines are arranged side by side or circle patterns are arranged on concentric circles.

The diffraction grating can be configured by, for example, forming a large number of parallel lines or concentric circle lines of grooves or patterns having different refractive indexes on a glass plate or the like. A pitch of the patterns is set according to conditions such as a distance to the indicator layer 17 and a maximum diffraction order of diffracted light in use. However, the pitch is in a range of 0.1 µm to 50 µm and more desirably in a range of 0.5 µm to 10 µm.

When the diffraction grating receives the excitation light E outputted from the LED element 15C, the diffraction grating diffuses the excitation light E into zero-th order, first order, second order, or higher order diffracted light according to diffraction. The higher order diffracted light spreads with an angle changed larger from an angle before penetration into the diffraction grating. Since the light spreads, a light amount distribution of the penetrating excitation light becomes uniform compared with a light amount distribution before the penetration into the diffraction grating. In order to block the zero-th diffracted light, a reflective film or the like same as the reflective film 20 is disposed.

The light amount uniformizing section having the scattering function for diffusing incident light according to a scattering phenomenon is a resin layer including particles having a diameter of, for example, about 1 nm to 10 µm. The particles may be opaque or may be transparent as long as the particles have a refractive index different from a refractive index of resin.

As a scattering effect, any phenomenon such as Rayleigh scattering or Mie scattering may be used as long as light scatters.

The light amount uniformizing section having the refracting function for diffusing incident light according to a refracting phenomenon includes, for example, a concave lens, a Fresnel lens, a micro concave lens array, or a prism array. The structure is formed by performing patterning on a transparent film such an oxide film with wet etching, machining transparent resin in a desired shape, or machining the second principal plane 15B.

For example, in a fluorescence sensor 10A according to the modification shown in Fig. 8, a concave section is formed on the second principal plane 15B of the LED substrate 15. The transparent resin layer 16 having a refractive index different from a refractive index of the LED substrate 15 is embedded in the concave section, whereby an excitation light diffusing section 21 having a concave lens function is formed. The excitation light diffusing section 21 is a part of the transparent resin layer 16 and is also a part of the LED substrate 15 formed by machining the second principal plane 15B. It goes without saying that a separately manufactured concave lens may be disposed on the LED substrate 15.

Excitation light E4 generated by the LED element 15C is diffused to an outer peripheral side by the excitation light diffusing section 21 having the concave lens function. Therefore, a light amount in a center region decreases and a light amount in a peripheral region increases.

For example, the Fresnel lens diffuses excitation light outputted from the LED element 15C in a wider angle according to refraction. By designing the Fresnel lens such that a refraction angle is larger in a center region having a larger light amount, it is possible to make a light amount distribution of the excitation light intruding into the indicator layer 17 more uniform.

When the micro lens array receives excitation light, respective lenses of the micro lens array diffuse the excitation light. A degree of refraction depends on the focal lengths of the lenses. Since the focal lengths of the lenses can be separately set, by increasing a refraction angle of the lens disposed in a center region where a light amount is large, it is possible to make a light amount distribution of the excitation light intruding into the indicator layer 17 more uniform.

When the prism array receives excitation light, respective prisms of the prism array diffuse the excitation light in arbitrary directions. For example, by refracting light in the center region having a large amount of light at a large angle, it is possible to make a light amount distribution of the excitation light intruding into the indicator layer 17 more uniform.

All the excitation light diffusing sections that average a light amount distribution may be disposed on the second principal plane 15B of the LED substrate 15 after formation or may be formed by machining the second principal plane 15B.

### Second Embodiment

A fluorescence sensor 10B according to a second embodiment is explained. Since the fluorescence sensor 10B is similar to the fluorescence sensor 10, components having the same functions are denoted by the same reference numerals and signs and explanation of the components is omitted. A light amount uniformizing section of the fluorescence sensor 10B is a light blocking film 22 having a function of absorbing excitation light formed in at least a region of the second principal plane 15B opposed to the LED element 15C. The light blocking film 22 is an excitation light attenuating section that attenuates a light amount of a region where a light amount of a light amount distribution is large.

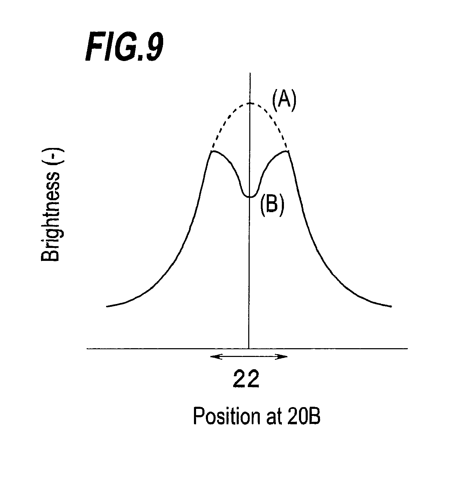

As shown in Fig. 9, compared with a case (A) in which a light blocking film is absent, in a case (B) in which a light blocking film is present, although a light amount in a center region decreases, a light amount in a peripheral region does not change. The light blocking film 22 may be disposed only in a region where a light amount of excitation light is large, i.e., the excitation light is intense or a stronger effect of the light blocking film 22 may be produced in a region where the excitation light is more intense. For example, the thickness of the light blocking film 22 may be changed such that absorption is the largest in a center and is the smallest in a periphery. Like the reflective film patterns 20D shown in Fig. 5D, the light blocking film 22 may include plural patterns.

For example, opaque resin having excitation light transmittance of about 10% to 90% may be used in the light blocking film 22 or the light blocking film 22 may be dot patterns or stripe patterns of an opaque film.

The light blocking film 22 is easily manufactured. However, by attenuating excitation light in a place where light emission intensity of the excitation light is high, the light blocking film 22 can uniformize a light amount received by the indicator layer 17. Therefore, like the fluorescence sensor 10 and the like, the fluorescence sensor 10B has long life.

### Third Embodiment

A fluorescence sensor 10C according to a third embodiment is explained. Since the fluorescence sensor 10C is similar to the fluorescence sensor 10, components having the same functions are denoted by the same reference numerals and signs and explanation of the components is omitted.

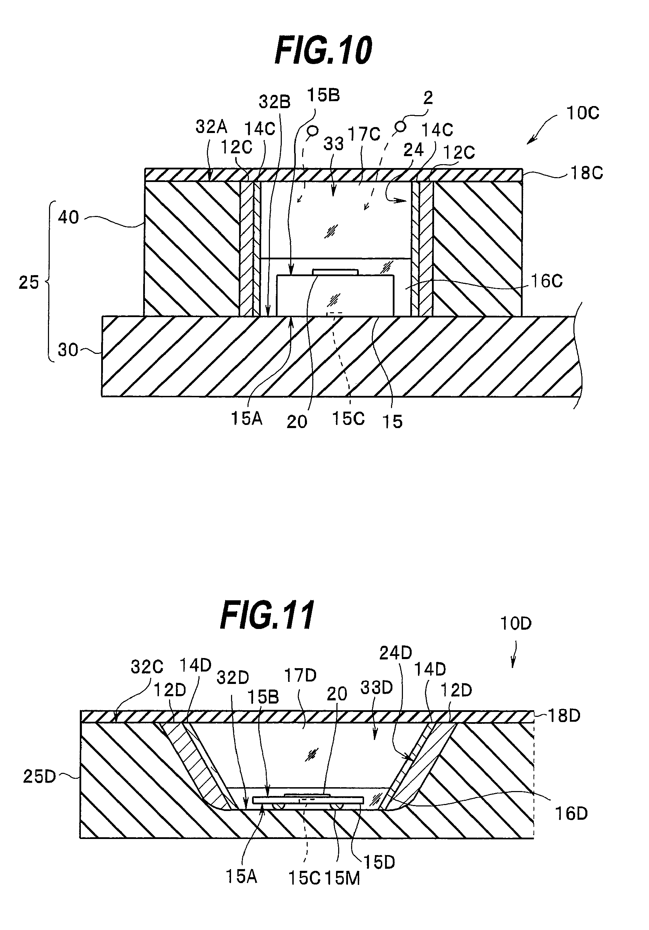

As shown in Fig. 10, the fluorescence sensor 10C includes a main substrate 25 on which a concave section 33 is formed, the LED substrate 15 on which the reflective film 20 is disposed, a transparent resin layer 16C, an indicator layer 17C, and a light blocking layer 18C. The LED substrate 15, the transparent resin layer 16C, and the indicator layer 17C are disposed on an inside of the concave section 33. The light blocking layer 18C is disposed to close an opening of the concave section 33.

The main substrate 25 is manufactured by joining a wiring substrate 30 including not-shown various wiring layers and a frame-shaped substrate 40 formed in a square pole shape including a through-hole in a center. Therefore, in the main substrate 25, the concave section 33 having a bottom surface 32B parallel to a first principal plane 32A is present on the first principal plane 32A. A surface of the wiring substrate 30 is the bottom surface 32B of the concave section 33 and inner walls of the through-hole of the frame-shaped substrate 40 are inner walls 24 of the concave section 33. In the fluorescence sensor 10C, both of an external shape of the frame-shaped substrate 40 and an inner surface shape of the concave section 33 are rectangular parallelepipeds but may be square poles, prisms, or columns.

PD elements 12C are formed on four inner walls of the through-hole of the frame-shaped substrate 40, i.e., four inner walls 24 of the concave section 33 of the main substrate 25. For improvement of detection sensitivity, the PD elements 12C are desirably formed on all the four inner walls surrounding the indicator layer 17C. However, the PD element 12C only has to be formed at least in a part of at least one inner wall.

To form the PD elements 12C on the wall surfaces 24, ion injection processing is performed from four directions in a state in which a substrate made of a semiconductor such as silicon is tilted at 5 degrees to 30 degrees. For example, conditions in injecting boron are an acceleration voltage of about 10 keV to 100 keV and an injection amount of about 1×10¹²cm⁻² to 5×10¹⁵cm⁻².

Filters 14C are disposed to cover the PD elements 12C formed on the four inner walls 24. The filters 14C block the excitation light E and transmits the fluorescence F having wavelength larger than wavelength of the excitation light E.

The LED substrate 15 is disposed on the bottom surface 32B of the concave section 33 with the second principal plane 15B faced up. The indicator layer 17C is disposed on the transparent resin layer 16C that covers the LED substrate 15.

As in the fluorescence sensor 10 according to the first embodiment, the reflective film 20 is a reflective filter having an interference effect and is a light amount uniformizing section that uniformizes a light amount distribution of the excitation light E radiated from the second principal plane 15B. It goes without saying that, as the light amount uniformizing section, the various excitation light diffusing sections or the various excitation light attenuating sections explained above may be used.

Operations of the fluorescence sensor 10C are explained.

The excitation light E radiated from the second principal plane 15B of the LED substrate 15 is averaged by the reflective film 20 and made incident on the indicator layer 17C via the transparent resin layer 16C. The indicator layer 17C emits the fluorescence F having intensity corresponding to an analyte amount.

The fluorescence F generated by the indicator layer 17C is made incident on the PD elements 12C, which is formed on the inner walls 24, via the filters 14C. A signal corresponding to intensity of the fluorescence F is outputted from the PD elements 12C. The excitation light E is blocked by the filters 14C and is not made incident on the PD elements 12C.

As explained above, in the fluorescence sensor 10C, the concave section 33 having the bottom surface 32B parallel to the principal plane 32A is present on the main substrate 25. The PD elements 12C are formed on the inner walls 24 of the concave section 33. The LED substrate 15 and the indicator layer 17C are disposed on the inside of the concave section 33. In particular, the fluorescence sensor 10C includes the main substrate 25 in which the wiring substrate 30 made of a semiconductor such as silicon and the frame-shaped substrate 40 including the through-hole are joined.

The fluorescence sensor 10C has effects same as the effects of the fluorescence sensor 10 according to the first embodiment. Further, since the fluorescence F is detected by the PD elements 12C formed on the inner walls 24 surrounding the indicator layer 17, the fluorescence sensor 10C has high detection sensitivity.

The LED substrate 15 having the functions of the wiring substrate 30 may be joined to cover a lower surface of the through-hole of the frame-shaped substrate 40. As explained below, a wall surface of the through-hole may be formed in a tapered shape.

### Modification of the Third Embodiment

A fluorescence sensor 10D according to a modification of the third embodiment of the present invention is explained. Since the fluorescence sensor 10D is similar to the fluorescence sensor 10C according to the third embodiment, the same components are denoted by the same reference numerals and signs and explanation of the components is omitted.

As shown in Fig. 11, in the fluorescence sensor 10D, a main substrate 25D equivalent to the wiring substrate 30 and the frame-shaped substrate 40 according to the third embodiment is integrally formed by a semiconductor substrate, for example, a silicon substrate. A concave section 33D of the main substrate 25D is a rectangular concave section formed on a first principal plane 32C of the silicon substrate by, for example, an etching method.

As the etching method, wet etching methods for performing etching using a tetramethylammonium (TMAH) hydroxide water solution, a potassium hydroxide (KOH) water solution, and the like are desirable. However, dry etching methods such as reactive ion etching (RIE) and chemical dry etching (CDE) can also be used.

For example, in the case of a silicon substrate having a principal plane with a plane orientation of (100) plane, anisotropic etching having low etching speed of a (111) plane compared with the (100) plane is performed. Therefore, the inner walls 24D of the concave section 33D are (111) planes. An angle θ1 formed with the (100) plane is 54.74 degrees. In other words, four inner walls 24D of the concave section 33D having an opening and a bottom surface 32D which are rectangular, are formed in a tapered shape.

PD elements 12D are formed on the inner walls 24D of the concave section 33D. The PD elements 12D are formed at least on a part of at least one inner wall. The concave section 33D having a taper in the inner walls has a wide area for formation of the PD elements compared with the concave section 33 having the vertical inner wall 24. Moreover, formation of the PD elements 12D on the inner walls 24D is easy. After the formation of the PD elements 12D, filters 14D are disposed to cover the PD elements 12D.

Depending on specifications of a fluorescence sensor, as in the fluorescence sensor 10C, the wall surfaces 24D of the concave section 33D may be perpendicular to the principal plane. A PD element may be formed on the bottom surface 32D of the concave section 33D and a filter may be disposed to cover the PD element.

In the fluorescence sensor 10D, an LED substrate 15D is polished from the second principal plane 15B side and thinned to thickness of, for example, 10 µm. Therefore, compared with a fluorescence sensor in which an un-thinned LED substrate is disposed, since thickness occupied by an indicator layer 17D with respect to depth of the concave section 33D is large, a larger amount of fluorescence is generated. Therefore, the fluorescence sensor 10D has high sensitivity. A fluorescence senor having a thinned LED substrate can obtain sufficient detection sensitivity even if a concave section is shallow.

On the second principal plane 15B of the LED substrate 15D, the reflective film 20 functioning as the light amount uniformizing section same as the reflective film 20 of the fluorescence sensor 10 according to the first embodiment is disposed. It goes without saying that, as the light amount uniformizing section, the various excitation light diffusing sections or the various excitation light attenuating sections explained above may be used.

In Fig. 11, as in the first embodiment, wires and the like connected to electrodes 15M of the LED element 15C formed on the first principal plane 15A of the LED substrate 15D are not shown. After the transparent resin layer 16D is disposed in the concave section 33D to cover the LED substrate 15D, the indicator layer 17D is disposed on an inside of the concave section 33D. A light blocking layer 18D is disposed to close an opening of the concave section 33D.

In the fluorescence sensor 10D, the concave section 33D including the bottom surface 32D parallel to the principal plane 32C is present on the main substrate 25D made of a semiconductor. The PD elements 12D are formed on the inner walls 24D of the concave section 33D. The LED substrate 15D and the indicator layer 17D are disposed on the inside of the concave section 33D. In particular, in the fluorescence sensor 10D, the concave section 33D is formed on the main substrate 25D made of silicon by etching.

The fluorescence sensor 10D has the effects of the fluorescence sensor 10C and the like. Further, the fluorescence sensor 10D is easily manufactured and suitable for a reduction in size and has higher sensitivity.

## Claims

1. A fluorescence sensor comprising:
a main substrate on which a photoelectric conversion element that converts fluorescence into an electric signal is formed;
a light-emitting element substrate having a first principal plane on which a light-emitting element that generates excitation light is formed;
a light amount uniformizing section that uniformizes a light amount distribution of the excitation light radiated from a second principal plane of the light-emitting element substrate; and
an indicator that receives the excitation light uniformized by the light amount uniformizing section and generates the fluorescence having a light amount corresponding to an analyte amount.

2. The fluorescence sensor according to claim 1, wherein
a concave section including a bottom surface parallel to a principal plane is present on the main substrate,
the photoelectric conversion element is formed on an inner wall of the concave section, and
the light-emitting element substrate and the indicator are disposed on an inside of the concave section.

3. The fluorescence sensor according to claim 1, wherein
a through-hole is present in the main substrate,
the photoelectric conversion element is formed on an inner wall of the through-hole, and
the indicator is disposed on an inside of the through-hole.

4. The fluorescence sensor according to any one of claims 1 to 3, wherein the light amount uniformizing section is an excitation light diffusing section that averages the light amount distribution.

5. The fluorescence sensor according to claim 4, wherein the excitation light diffusing section has a diffracting function, a scattering function, a refracting function, or a reflecting function.

6. The fluorescence sensor according to claim 5, wherein the excitation light diffusing section is a reflective film formed in at least a region of the second principal plane opposed to the light-emitting element.

7. The fluorescence sensor according to claim 6, wherein the reflective film is a multilayer film that reflects the excitation light and transmits the fluorescence.

8. The fluorescence sensor according to any one of claims 1 to 3, wherein the light amount uniformizing section is an excitation light attenuating section that attenuates a light amount of a region where a light amount of the excitation light is large.

9. The fluorescence sensor according to claim 8, wherein the excitation light attenuating section is a light blocking film that is formed in at least a region of the second principal plane opposed to the light-emitting element and has a function of absorbing the excitation light.
